# EUROPÄISCHE PATENTSCHRIFT

(11) **EP 1 696 863 B1**
(45) Veröffentlichungstag und Bekanntmachung des Hinweises auf die Patenterteilung: **12.11.2008**
(21) Anmeldenummer: 04797886.1
(22) Anmeldetag: 13.11.2004
(51) Int. Cl.: A61K 8/03, A61Q 5/04, A61K 8/44

(54) **ZWEI-ODER MEHRPHASIGE KOSMETISCHE MITTEL MIT VERBESSERTEM REVERSIBLEN MISCHUNGS-UND ENTMISCHUNGSVERHALTEN**
DUAL OR MULTIPLE PHASE COSMETIC AGENT WITH IMPROVED REVERSIBLE MIXING AND SEPARATION BEHAVIOUR
AGENTS COSMETIQUES A DEUX OU PLUSIEURS PHASES PRESENTANT UN MEILLEUR COMPORTEMENT DE MELANGE ET DE SEPARATION REVERSIBLE

(30) Priorität: 19.12.2003 DE 10360688
(43) Veröffentlichungstag der Anmeldung: 06.09.2006
(73) Patentinhaber: Henkel AG & Co. KGaA, 40589 Düsseldorf (DE)
(72) Erfinder: RAUTENBERG-GROTH, Birgit, 25479 Ellerau (DE); KNAPPE, Thorsten, 22869 Schenefeld (DE); MÜLLER, Burkhard, 21075 Hamburg (DE)
(86) Internationale Anmeldenummer: PCT/EP2004/012906
(87) Internationale Veröffentlichungsnummer: WO 2005/063177

(56) Entgegenhaltungen:
- WO-A-02/15849
- WO-A-94/04125
- WO-A-99/58099
- WO-A-02/067881
- "Rohstoffinformation zu Protelan VE/K"

## Beschreibung

Die Erfindung betrifft ein Verfahren zur dauerhaften Verformung von Keratinfasern, insbesondere von menschlichen Haaren, durch reduktive Spaltung und erneute oxidative Knüpfung von Disulfidbindungen des Keratins sowie für dieses Verfahren geeignete mehrphasige Mittel mit verbessertem Mischungs- und Entmischungsverhalten.

Die dauerhafte Verformung von Keratinfasern wird üblicherweise so durchgeführt, daß man die Faser mechanisch verformt und die Verformung durch geeignete Hilfsmittel festlegt. Vor und/oder nach dieser Verformung behandelt man die Faser mit der wäßrigen Zubereitung einer keratinreduzierenden Substanz und spült nach einer Einwirkungszeit mit Wasser oder einer wäßrigen Lösung. In einem zweiten Schritt behandelt man dann die Faser mit der wäßrigen Zubereitung eines Oxidationsmittels. Nach einer Einwirkungszeit wird auch dieses ausgespült und die Faser von den mechanischen Verformungshilfsmitteln (Wickler, Papilloten) befreit.

Die wäßrige Zubereitung des Keratinreduktionsmittels ist üblicherweise alkalisch eingestellt, damit zum einen genügender Anteil der Thiolfunktionen deprotoniert vorliegt und zum anderen die Faser quillt und auf diese Weise ein tiefes Eindringen der keratinreduzierenden Substanz in die Faser ermöglicht wird. Die keratinreduzierende Substanz spaltet einen Teil der Disulfid-Bindungen des Keratins zu -SH-Gruppen, so daß es zu einer Lockerung der Peptidvernetzung und infolge der Spannung der Faser durch die mechanische Verformung zu einer Neuorientierung des Keratingefüges kommt. Unter dem Einfluß des Oxidationsmittels werden erneut Disulfid-Bindungen geknüpft, und auf diese Weise wird das Keratingefüge in der vorgegebenen Verformung neu fixiert. Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.

Eine negative Begleiterscheinung der so durchgeführten Dauerwellung des Haares ist aber häufig ein Verspröden und Stumpfwerden der Haare. Weiterhin werden in vielen Fällen auch andere Eigenschaften wie Naß- und Trockenkämmbarkeit, Griff, Geschmeidigkeit, Weichheit, Glanz und Reißfestigkeit in ungewünschter Weise beeinflußt.

Es hat daher in der Vergangenheit nicht an Versuchen gefehlt, hier für Abhilfe zu sorgen.

Eine entsprechende Modifizierung der reduzierenden Lösung führt zu in der Regel nicht befriedigenden Welleistungen. Die Zugabe bekannter Zusätze wie Strukturanten, Polymere, Filmbildner und vernetzende Harze oder die neutrale bis schwach saure Einstellung der Zubereitung kann die Schädigung des Haares zwar verringern, jedoch bleibt das Haar in seiner Struktur mehr oder weniger geschwächt. Die pflegende Behandlung der Haare durch weitere Nachbehandlungen kann zwar die Haareigenschaften wieder verbessern, erfordert jedoch zusätzlichen Zeitaufwand und in der Regel die Verwendung mindestens eines weiteren Mittels.

Es bestand daher weiterhin die Aufgabe, ein Verfahren der dauerhaften Verformung von Keratinfasern zu finden, bei welchem die genannten unerwünschten Nebenwirkungen weiter reduziert oder ganz ausgeschlossen werden.

Es wurde bereits in der Patentanmeldung WO-A1-99/58099 offenbart, daß eine wesentliche Verbesserung der Eigenschaften verformter Keratinfasern, wie verbesserte Kämmbarkeit und Avivage, dadurch erreicht wird, daß mindestens ein während des Verformungsverfahrens eingesetztes Mittel zwei- oder mehrphasig formuliert ist und spezielle Verbindungen enthält. Ferner ist aus der Offenlegungsschrift WO-A2-02/67881 bekannt, dass sich die Kämmbarkeit und Avivage durch den Einsatz von Silikonölen mit ausgewählter kinematischer Viskosität in solchen zwei- oder mehrphasigen Mitteln weiter verbessern lassen.

Zur Verwirklichung einer gleichbleibend effektiven Wirkung eines Zwei- oder Mehrphasenmittels ist einerseits die homogene Mischung der Phasen vor der Anwendung, sowie andererseits die Reversibilität dieser Mischung entscheidend. Allerdings ist in den mehrphasigen Mitteln des Standes der Technik das Misch- und das Entmischungsverhalten der Phasen nach der Anwendung nicht befriedigend. Insbesondere bei mehrmalig ausgeführtem Misch- und Entmischvorgang ist die vollständige Mischung bzw. insbesondere Entmischung der Phasen nicht zufriedenstellend gewährleistet.

Gegenstand der Erfindung ist daher ein Verfahren zur dauerhaften Verformung von Keratinfasern, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült und gegebenenfalls nachbehandelt, dadurch gekennzeichnet, daß mindestens eine der beiden wäßrigen Zubereitungen oder die erste Spülung in Form eines Zwei- oder Mehrphasensystems, enthaltend mindestens zwei separate, Kontinuierliche Phasen, vorliegt, das mindestens eine Ölkomponente und als Emulgator mindestens eine N-Acyl-glutaminsäure oder deren Salze enthält und das zum Aufbringen auf die Faser durch mechanische Bewegung reversibel in ein homogenes System überführt wird.

Im weiteren werden folgende Bezeichnungen verwendet:
- "Wellmittel" für die wäßrige Zubereitung der keratinreduzierenden Substanz,
- "Zwischenspülung" für die erste Spülung und
- "Fixiermittel" für die wäßrige Zubereitung des Oxidationsmittels.

Im erfindungsgemäßen Verfahren ist das Wellmittel, die Zwischenspülung und/oder das Fixiermittel in Form eines Zwei- oder Mehrphasensystems formuliert. Erfindungsgemäß eingesetzte Zwei- und Mehrphasensysteme sind Systeme, bei denen mindestens zwei separate, kontinuierliche Phasen vorliegen. Beispiele für solche Systeme sind Zubereitungen, die folgende Phasen aufweisen:
- eine wäßrige Phase und eine nichtwäßrige Phase, die separat voneinander vorliegen
- eine wäßrige Phase und zwei nichtwäßrige, miteinander nicht mischbare Phasen, die jeweils separat vorliegen
- eine Öl-in-Wasser-Emulsion und eine davon separiert vorliegende nichtwäßrige Phase
- eine Wasser-in-Öl-Emulsion und eine davon separiert vorliegende wäßrige Phase.

Keine Zwei-Phasensysteme im Sinne der vorliegenden Erfindung sind Systeme, bei denen nur eine kontinuierliche Phase vorliegt, wie z. B. reine Öl-in-Wasser- oder Wasser-in-Öl-Emulsionen.

Die im erfindungsgemäßen Verfahren eingesetzten, Zwei- oder Mehrphasensysteme aufweisenden Mittel entfalten ihre volle Wirkung aber nur dann, wenn sie in homogener Form auf die Keratinfaser aufgebracht werden. Dazu werden die Mittel durch mechanische Einwirkung, z. B. einfaches Schütteln des sie enthaltenden Behälters mit der Hand, reversibel in homogene Systeme überführt. Um ein homogenes Auftragen auf die Keratinfaser sicherzustellen, muß dieser homogene Zustand für eine ausreichende Zeit erhalten bleiben, bevor sich die einzelnen Phasen wieder ausbilden. Für die erfindungsgemäße Lehre hat es sich als ausreichend erwiesen, wenn dieser homogene Zustand mindestens 20 Sekunden, insbesondere mindestens 30 Sekunden, stabil ist, bevor für den Betrachter wieder eine Grenzschicht und somit die Ausbildung der einzelnen Phasen erkennbar wird. Nach einem gewissen Zeitraum, bevorzugt von höchstens 60 Minuten, besonders bevorzugt von höchstens 30 Minuten, hat sich die reversible Mischung der Phasen durch Entmischung vollzogen, so dass der Ausgangszustand unter Erhalt des Zwei- oder Mehrphasensystems wieder erreicht wird.

Die erfindungsgemäß eingesetzten Zwei- und Mehrphasensysteme enthalten neben Wasser zwingend mindestens eine Ölkomponente.

Erfindungsgemäß geeignete Ölkomponenten sind prinzipiell alle Öle und Fettstoffe sowie deren Mischungen mit festen Paraffinen und Wachsen. Bevorzugt sind solche Ölkomponenten, deren Löslichkeit in Wasser bei 20 °C kleiner 1 Gew.-%, insbesondere kleiner als 0,1 Gew.-% beträgt. Der Schmelzpunkt der einzelnen Öl- oder Fettkomponenten liegt bevorzugt unterhalb von etwa 40 °C. Ölkomponenten, die bei Raumtemperatur, d. h. unterhalb von 25 °C flüssig sind, können erfindungsgemäß besonders bevorzugt sein. Bei Verwendung mehrerer Öl- und Fettkomponenten sowie ggf. festen Paraffinen und Wachsen ist es in der Regel jedoch auch ausreichend, wenn die Mischung der Öl- und Fettkomponenten sowie ggf. Paraffine und Wachse diesen Bedingungen genügt.

Die Ölkomponenten sind bevorzugt im Gewichtsverhältnisbereich zu den restlichen Komponenten von 1 zu 200 bis 1 zu 1, besonders bevorzugt von 1 zu 40 bis 1 zu 1, ganz besonders bevorzugt von 1 zu 20 bis 1 zu 3, in den Mitteln des erfindungsgemäßen Verfahrens enthalten.

Eine bevorzugte Gruppe von Ölkomponenten sind pflanzliche Öle. Beispiele für solche Öle sind Aprikosenkernöl, Avokadoöl, Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.

Eine weitere, besonders bevorzugte Gruppe erfindungsgemäß einsetzbarer Ölkomponenten sind flüssige Paraffinöle und synthetische Kohlenwasserstoffe sowie Din-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.

Ebenfalls erfindungsgemäß einsetzbare Ölkomponenten sind Fettsäure- und Fettalkoholester. Bevorzugt sind die Monoester der Fettsäuren mit Alkoholen mit 3 bis 24 C-Atomen. Bei dieser Stoffgruppe handelt es sich um die Produkte der Veresterung von Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Isopropylalkohol, Glycerin, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol^{®} SN), Stearinsäure-2-ethylhexylester (Cetiol^{®} 868), Cetyloleat, Glycerintricaprylat, Kokosfettalkohol-caprinat/-caprylat und n-Butylstearat.

Weiterhin stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglykoldioleat, Ethylenglykol-di-isotridecanoat, Propylenglykol-di(2-ethylhexanoat), Propylenglykol-di-isostearat, Propylenglykol-di-pelargonat, Butandiol-di-isostearat und Neopentylglykoldi-capylat erfindungsgemäß verwendbare Ölkomponenten dar, ebenso komplexe Ester wie z. B. das Diacetyl-glycerinmonostearat.

Erfindungsgemäß bevorzugt verwendbare hydrophobe Komponenten sind schließlich auch Silikonöle, insbesondere Dialkyl- und Alkylarylsiloxane, wie beispielsweise Dimethylpolysiloxan und Methylphenylpolysiloxan, sowie deren alkoxylierte und quaternierte Analoga sowie cyclische Siloxane. Beispiele für solche Silikone sind die von Dow Corning unter den Bezeichnungen DC 190, DC 200 und DC 1401 vertriebenen Produkte sowie die Handelsprodukte DC 344 und DC 345 von Dow Corning, Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80). Bevorzugt im Sinne der Erfindung können Silikonöle mit einer kinematischen Viskosität bis zu 50.000 cSt gemessen bei 25 °C sein. Ganz besonders bevorzugt sind Silikonöle mit kinematischen Viskositäten bis zu 10.000 cSt gemessen bei 25 °C. Die Bestimmung der Viskositäten erfolgt dabei nach der Kugelfallmethode entsprechend der Methode "british standard 188". Vergleichbare Werte werden mit zum "british standard 188" analogen Prüfvorschriften der Hersteller erhalten, beispielsweise der "CTM 0577" der Dow Corning Corporation.

In einer besonderen Ausführungsform werden als hydrophobe Komponente insbesondere cyclische Siloxane wie beispielsweise die Produkte Dow Corning^{®} 344, Dow Corning^{®} 345, Dow Corning^{®} 244, Dow Corning^{®} 245 oder Dow Corning^{®} 246 mit kinematischen Viskositäten von bis zu 10.000 cSt bei 25 °C bestimmt entsprechend den Angaben des Herstellers eingesetzt.

Die zwingend in den zwei- oder mehrphasigen Mitteln des erfindungsgemäßen Verfahrens als Emulgator enthaltene N-Acyl-glutaminsäure bzw. deren Salze wird besonders bevorzugt in Kombination mit einem Silikonöl als Ölkomponente in den zwei-oder mehrphasigen Mitteln des erfindungsgemäßen Verfahrens eingesetzt.

Erfindungsgemäß einsetzbare Ölkomponenten sind schließlich auch Dialkylcarbonate, wie sie in der DE-OS 197 101 54, auf die ausdrücklich Bezug genommen wird, ausführlich beschrieben werden. Dioctylcarbonate, insbesondere das Di-2-ethylhexylcarbonat, sind bevorzugte Ölkomponenten in Rahmen der vorliegenden Erfindung.

Die in den zwei- oder mehrphasigen Mitteln des erfindungsgemäßen Verfahrens enthaltene N-Acyl-glutaminsäure ist ein Kondensationsprodukt aus Glutaminsäure mit gesättigten oder einfach oder mehrfach ungesättigten Fettsäuren bzw. Fettsäuregemischen, wie beispielsweise Ölsäure, Myristinsäure, Laurinsäure, Caprinsäure, Palmitinsäure, Undecylensäure, Kokosfettsäure und Abietinsäure. Diese Kondensationsprodukte können auch in Form von Salzen, insbesondere Natrium-, Kalium-, Magnesium- und Triethanolaminsalzen, vorliegen.

Es ist erfindungsgemäß bevorzugt, wenn in den zwei- oder mehrphasigen Mitteln die N-Acyl-glutaminsäure ausgewählt ist aus N-(C₈-C₃₀)-Acyl-glutaminsäure sowie deren Salze.

Beispiele für im Handel erhältliche erfindungsgemäße N-Acyl-glutaminsäuren bzw. deren Salze sind Plantapon^{®} ACG 35 (Cognis Deutschland), Protelan^{®} AG 8 (Zschimmer & Schwarz Italiana), Protelan^{®} AG 100 (Zschimmer & Schwarz Italiana), Protelan^{®} AG 110/N (Zschimmer & Schwarz Italiana), Sepifeel One^{®} (Seppic), Protelan^{®} AG 89/K (Zschimmer & Schwarz Italiana), Amisoft^{®} ECS-22 (Ajinomoto USA), Amisoft^{®} LS-22 (Ajinomoto USA), Amisoft^{®} HS-22 (Ajinomoto USA) und Acylglutamate^{®} MK-11 (Ajinomoto USA).

Die N-Acyl-glutaminsäure bzw. deren Salze ist in den Mitteln bevorzugt in einer Menge von 0.05 bis 10 Gew.%, insbesondere von 0.1 bis 2 Gew.%, bezogen auf das Gewicht des Mittels, enthalten.

Die N-Acyl-glutaminsäure bzw. deren Salze ist bevorzugt in einem Gewichtsverhältnis zur Ölkomponente von 1 zu 200 bis 1 zu 7, insbesondere von 1 zu 100 bis 1 zu 5, in den Mitteln enthalten.

Erfindungsgemäß können zusätzlich zu den genannten Ölkomponenten mit Wasser nur begrenzt mischbare Alkohole eingesetzt werden.

Unter "mit Wasser begrenzt mischbar" werden solche Alkohole verstanden, die in Wasser bei 20 °C zu nicht mehr als 10 Gew.-%, bezogen auf die Wassermasse, löslich sind.

In vielen Fällen haben sich Triole und insbesondere Diole als erfindungsgemäß besonders geeignet erwiesen. Erfindungsgemäß einsetzbar sind Alkohole mit 4 bis 20, insbesondere 4 bis 10, Kohlenstoffatomen. Die erfindungsgemäß verwendeten Alkohole können gesättigt oder ungesättigt und linear, verzweigt oder cyclisch sein. Einsetzbar im Sinne der Erfindung sind beispielsweise Butanol-1, Cyclohexanol, Pentanol-1, Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen.

Erfindungsgemäß bevorzugt als Alkohole sind 2-Ethyl-hexandiol-1,3, Butanol-1, Cyclohexanol, Pentanol-1 und 1,2-Butandiol. Insbesondere 2-Ethyl-hexandiol-1,3, aber auch Butanol-1 und Cyclohexanol sind besonders bevorzugt.

Die erfindungsgemäße Lehre umfaßt auch solche Ausführungsformen des erfindungsgemäßen Verfahrens, bei denen das mehrphasige Mittel aus zwei oder mehr getrennt konfektionierten Ausgangszubereitungen erst unmittelbar vor der Anwendung hergestellt wird. Diese Ausführungsform kann bei extrem inkompatiblen Bestandteilen bevorzugt sein.

Gemäß einer bevorzugten Ausführungsform des erfindungsgemäßen Verfahrens ist die Wellotion in Form des oben genannten Zwei- oder Mehrphasensystems formuliert. Es hat sich überraschenderweise gezeigt, daß auf diese Weise formulierte Wellotionen bei gleicher Menge der jeweiligen keratinreduzierenden Komponenten einen deutlich erhöhten Welleffekt ergeben. Analog kann die mit einer nicht erfindungsgemäß formulierten Wellotion erzielte Welleistung mit einer erfindungsgemäß formulierten Wellotion unter deutlicher Verringerung des Anteils der keratinreduzierenden Substanz, und somit unter Schonung von Haar und Kopfhaut, erreicht werden.

Weiterhin hat sich gezeigt, daß durch Formulierung der Wellotion als Zwei- und Mehrphasensystem das Problem der Parfümierung deutlich verringert werden kann. Aufgrund der von den meisten Anwendern nicht tolerierten Duftnote der in der Wellotion zwingend erforderlichen Bestandteile (keratinreduzierende Thio-Verbindungen, ggf. Alkalien wie Ammoniak oder Alkanolamine) ist eine Parfümierung jedoch praktisch unerläßlich. Problematisch ist, daß die überwiegende Zahl der Parfümkomponenten in diesen Wellotionen nicht lagerstabil ist. Somit ist die Auswahl der Duftnoten für solche Mittel stark eingeschränkt. Es hat sich nun ebenfalls überraschenderweise gezeigt, daß bei Verwendung der erfindungsgemäß definierten Zwei- oder Mehrphasensysteme eine Vielzahl weiterer Parfümkomponenten in diese Wellotion lagerstabil eingearbeitet werden kann. Auch in erfindungsgemäßen aufgebauten Fixierlösungen haben sich zusätzliche Parfümkomponenten als nun lagerstabil einarbeitbar erwiesen. Durch das verbesserte Entmischungsverhalten der unten genannten erfindungsgemäßen Mittel, die in dem erfindungsgemäßen Verfahren zur Anwendung kommen, wurde die Haltbarkeit der Parfumkomponente in zwei- oder mehrphasigen Mitteln weiterhin verbessert.

Bevorzugt wird das erfindungsgemäße Verfahren zum Dauerwellen bzw. Glätten von menschlichen Haaren verwendet.

Weitere Gegenstände der Anmeldung sind die zur Durchführung des erfindungsgemäßen Verfahrens verwendeten Mittel.

Diese Mittel dienen im Rahmen eines Verfahrens zur dauerhaften Verformung von Keratinfasern entweder zur Durchführung der reduzierenden Stufe, zur Durchführung der oxidierenden Stufe oder zum Spülen nach Durchführung der reduzierenden Stufe und können prinzipiell alle für diese Mittel üblichen Bestandteile enthalten, sofern die erfindungsgemäßen Bedingungen (Vorliegen des Zwei- oder Mehrphasensystems und die kurzzeitige Mischbarkeit) gegeben sind.

Ein zweiter Gegenstand der Erfindung ist daher ein Mittel zur Durchführung der reduzierenden Stufe eines Verfahrens zur dauerhaften Verformung von Keratinfasern enthaltend eine keratinreduzierende Substanz sowie übliche Bestandteile, dadurch gekennzeichnet, daß es in Form des obengenannks Zwei- oder Mehrphasensystems vorliegt, das mindestens eine Ölkomponente und als Emulgator N-Acyl-glutaminsäure oder deren Salze enthält und das durch mechanische Bewegung reversibel in ein homogenes System überführt werden kann.

Die erfindungsgemäßen Wellmittel enthalten zwingend die als keratinreduzierende Substanzen bekannten Mercaptane. Solche Verbindungen sind beispielsweise Thioglykolsäure, Thiomilchsäure, Thioäpfelsäure, Mercaptoethansulfonsäure sowie deren Salze und Ester, Cysteamin, Cystein, Bunte Salze und Salze der schwefligen Säure. Bevorzugt geeignet sind die Alkali- oder Ammoniumsalze der Thioglykolsäure und/oder der Thiomilchsäure sowie die freien Säuren. Diese werden in den Wellmitteln bevorzugt in Konzentrationen von 0,5 bis 1,0 mol/kg bei einem pH-Wert von 5 bis 12, insbesondere von 7 bis 9,5, eingesetzt. Zur Einstellung dieses pH-Wertes enthalten die erfindungsgemäßen Wellmittel üblicherweise Alkalisierungsmittel wie Ammoniak, Alkali- und Ammoniumcarbonate und -hydrogencarbonate oder organische Amine wie Monoethanolamin.

Weiterhin können die erfindungsgemäßen Wellotionen wellkraftverstärkende Komponenten enthalten, wie beispielsweise
- heterocyclische Verbindungen wie Imidazol, Pyrrolidin, Piperidin, Dioxolan, Dioxan, Morpholin und Piperazin sowie Derivate dieser Verbindungen wie beispielsweise die C₁₋₄-Alkyl-Derivate, C₁₋₄-Hydroxyalkyl-Derivate und C₁₋₄-Aminoalkyl-Derivate. Bevorzugte Substituenten, die sowohl an Kohlenstoffatomen als auch an Stickstoffatomen der heterocyclischen Ringsysteme positioniert sein können, sind Methyl-, Ethyl-, β-Hydroxyethyl- und β-Aminoethyl-Gruppen. Erfindungsgemäß bevorzugte Derivate heterocyclischer Verbindungen sind beispielsweise 1-Methylimidazol, 2-Methylimidazol, 4(5)-Methylimidazol, 1,2-Dimethylimidazol, 2-Ethylimidazol, 2-Isopropylimidazol, N-Methylpyrrolidon, 1-Methylpiperidin, 4-Methylpiperidin, 2-Ethylpiperidin, 4-Methylmorpholin, 4-(2-Hydroxyethyl)morpholin, 1-Ethylpiperazin, 1-(2-Hydroxyethyl)piperazin, 1-(2-Aminoethyl)piperazin. Weiterhin erfindungsgemäß bevorzugte Imidazolderivate sind Biotin, Hydantoin und Benzimidazol. Ganz besonders bevorzugt ist das Imidazol.
- Aminosäuren wie insbesondere Arginin, Citrullin, Histidin, Ornithin und Lysin. Die Aminosäuren können sowohl als freie Aminosäure als auch als Salze, z. B. als Hydrochloride, eingesetzt werden. Weiterhin haben sich auch Oligopeptide aus durchschnittlich 2-3 Aminosäuren, die einen hohen Anteil (> 50 %, insbesondere > 70 %) an den genannten Aminosäuren haben, als erfindungsgemäß einsetzbar erwiesen. Erfindungsgemäß besonders bevorzugt sind Arginin sowie dessen Salze und argininreiche Oligopeptide.
- Diole wie beispielsweise 2-Ethyl-1,3-hexandiol, 1,3-Butandiol, 1,4-Butandiol, 1,2-Propandiol, 1,3-Propandiol, Neopentylglykol und Ethylenglykol. 1,3-Diole, insbesondere 2-Ethyl-1,3-hexandiol und 1,3-Butandiol, haben sich als besonders gut geeignet erwiesen.

Bezüglich näherer Informationen zu solchen wellkraftverstärkenden Komponenten wird auf die Druckschriften DE-OS 44 36 065 und EP-B1-363 057 verwiesen, auf deren Inhalt ausdrücklich Bezug genommen wird.

Die wellkraftverstärkenden Verbindungen können in den erfindungsgemäßen Wellotionen in Mengen von 0,5 bis 5 Gew.-%, bezogen auf die gesamte Wellotion, enthalten sein. Mengen von 1 bis 4 Gew.-%, im Falle der Diole von 0,5 bis 3 Gew.-%, haben sich als ausreichend erwiesen, weshalb diese Mengen besonders bevorzugt sind.

Ein dritter Gegenstand der Erfindung ist ein Mittel zur Durchführung der oxidierenden Stufe eines Verfahrens zur dauerhaften Verformung von Keratinfasern enthaltend ein Oxidationsmittel sowie übliche Bestandteile, dadurch gekennzeichnet, daß es in Form des obengenannkn Zwei- oder Mehrphasensystems vorliegt, das mindestens eine Ölkomponente und als Emulgator mindestens eine N-Acyl-glutaminsäure oder deren Salze enthält und das durch mechanische Bewegung reversibel in ein homogenes System überführt werden kann.

Zwingender Bestandteil der erfindungsgemäßen Fixiermittel sind Oxidationsmittel, z. B. Natriumbromat, Kaliumbromat, Wasserstoffperoxid, und die zur Stabilisierung wäßriger Wasserstoffperoxidzubereitungen üblichen Stabilisatoren. Der pH-Wert solcher wäßriger H₂O₂-Zubereitungen, die üblicherweise etwa 0,5 bis 15 Gew.-%, gebrauchsfertig in der Regel etwa 0,5 bis 3 Gew.-%, H₂O₂ enthalten, liegt bevorzugt bei 2 bis 6, insbesondere 2 bis 4; er wird durch anorganische Säuren, bevorzugt Phosphorsäure, eingestellt. Fixiermittel auf Bromat-Basis enthalten die Bromate üblicherweise in Konzentrationen von 1 bis 10 Gew.-% eingesetzt und der pH-Wert der Lösungen wird auf 4 bis 7 eingestellt. Gleichfalls geeignet sind Fixiermittel auf enzymatischer Basis (z. B. Peroxidasen), die keine oder nur geringe Mengen an Oxidationsmitteln, insbesondere H₂O₂, enthalten.

Ein vierter Gegenstand der Erfindung ist ein Mittel zum Spülen nach Durchführung der reduzierenden Stufe eines Verfahrens zur dauerhaften Verformung von Keratinfasern enthaltend übliche Bestandteile, dadurch gekennzeichnet, daß es in Form eines Zwei-oder Mehrphasensystems vorliegt, das mindestens eine Ölkomponente und als Emulgator mindestens eine N-Acyl-glutaminsäure oder deren Salze enthält und das durch mechanische Bewegung reversibel in ein homogenes System überführt werden kann.

Es ist bevorzugt in den vorgenannten erfindungsgemäßen Mitteln die Ölkomponenten und N-Acyl-glutaminsäuren bzw. deren Salze sowie in den im ersten Gegenstand der Erfindung genannten Ausführungsformen einzusetzen.

Es kann erfindungsgemäß bevorzugt sein, in einer Ausführungsform der erfindungsgemäßen Mittel, die N-Acyl-glutaminsäure bzw. deren Salze als einzigen Emulgator in den erfindungsgemäßen Mitteln einzusetzen. Ferner kann es im Rahmen dieser Ausführungsform bevorzugt sein, die N-Acyl-glutaminsäure bzw. deren Salze als einzige N-Acyl-aminosäure in den erfindungsgemäßen Mitteln zu verwenden.

In einer weiteren Ausführungsform der vorgenannten zwei- oder mehrphasigen Mittel kann es bevorzugt sein, den Mitteln zusätzlich mindestens einen mit Wasser nur begrenzt mischbaren Alkohol zuzusetzen. Die bevorzugten Varianten dieser Ausführungsform werden bereits im ersten Gegenstand der Erfindung beschrieben.

Weiterhin hat es sich als vorteilhaft erwiesen, wenn die erfindungsgemäßen Mittel einen pflegenden Wirkstoff, ausgewählt aus Proteinhydrolysaten und deren Derivaten, enthalten.

Geeignete Proteinhydrolysate sind insbesondere Elastin-, Kollagen-, Keratin-, Milcheiweiß-, Seidenprotein-, Sojaprotein-, Mandelprotein-, Erbsenprotein-, Kartoffelprotein-, Haferprotein-, Maisprotein- und Weizenproteinhydrolysate. Dabei können Produkte auf pflanzlicher Basis erfindungsgemäß bevorzugt sein.

Geeignete Derivate sind insbesondere quaternisierte Proteinhydrolysate. Beispiele für diese Verbindungsklasse sind die unter den Bezeichnungen Lamequat^{®}L(CTFA-Bezeichnung: Lauryldimonium Hydroxypropylamino Hydrolyzed Animal Protein; Grünau), Croquat^{®}WKP und Gluadin^{®}WQ auf dem Markt befindlichen Produkte. Das letztgenannte Produkt, das auf pflanzlicher Basis beruht, kann bevorzugt sein.
Die Protein-Derivate sind in den erfindungsgemäßen Mitteln bevorzugt in Mengen von 0,1 bis 10 Gew.-%, bezogen auf die Gesamtmenge des Mittels enthalten. Mengen von 0,1 bis 5 Gew.-% sind bevorzugt.

Bevorzugt enthalten die erfindungsgemäßen Mittel weiterhin mindestens einen konditionierenden Wirkstoff.

Als konditionierende Wirkstoffe kommen bevorzugt kationische Polymere in Betracht. Dies sind in der Regel Polymere, die ein quartäres Stickstoffatom, beispielsweise in Form einer Ammoniumgruppe, enthalten.
Bevorzugte kationischen Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- Polysiloxane mit quaternären Gruppen,
- Polymere Dimethyldiallylammoniumsalze und deren Copolymere mit Estern und Amiden von Acrylsäure und Methacrylsäure. Die unter den Bezeichnungen Mer-quat^{®}100 (Poly(dimethyldiallylammoniumchlorid)) und Merquat^{®}550 (Dimethyldiallylammo-niumchlorid-Acrylamid-Copolymer) im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Vinylimidazoliniummethochlorid-Copolymere, wie sie unter der Bezeichnung Luviquat^{®} angeboten werden,
- quaternierter Polyvinylalkohol
sowie die unter den Bezeichnungen
- Polyquaternium 2,
- Polyquaternium 17,
- Polyquaternium 18 und
- Polyquaternium 27
bekannten Polymeren mit quartären Stickstoffatomen in der Polymerhauptkette.

Geeignet als konditionierende Wirkstoffe sind auch Ampho-Polymere. Unter dem Oberbegriff Ampho-Polymere sind amphotere Polymere, d. h. Polymere, die im Molekül sowohl freie Aminogruppen als auch freie -COOH- oder SO₃H-Gruppen enthalten und zur Ausbildung innerer Salze befähigt sind, zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten. Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt. Ebenfalls bevorzugte Amphopolymere setzen sich aus ungesättigten Carbonsäuren (z. B. Acryl- und Methacryl-säure), kationisch derivatisierten ungesättigten Carbonsäuren (z. B. Acrylamidopropyl-trimethyl-ammoniumchlorid) und gegebenenfalls weiteren ionischen oder nichtionogenen Monomeren zusammen, wie beispielsweise in der deutschen Offenlegungsschrift 39 29 973 und dem dort zitierten Stand der Technik zu entnehmen sind. Terpolymere von Acrylsäure, Methylacrylat und Methacrylamidopropyltrimoniumchlorid, wie sie unter der Bezeichnung Merquat^{®}2001 N im Handel erhältlich sind sowie das Handelsprodukt Merquat^{®} 280, sind erfindungsgemäß besonders bevorzugte Ampho-Polymere.

Die kationischen oder amphoteren Polymere sind in den erfindungsgemäßen Zubereitungen bevorzugt in Mengen von 0,1 bis 5 Gew.-%, bezogen auf die gesamte Zubereitung, enthalten.

Als konditionierende Wirkstoffe eignen sich weiterhin Silikon-Gums, wie z.B. das Handelsprodukt Fancorsil^{®} LIM-1, sowie anionische Silikone, wie beispielsweise das Produkt Dow Corning^{®}1784.

Beispiele für die in den erfindungsgemäßen Mitteln als konditionierende Wirkstoffe verwendbaren kationischen Tenside sind insbesondere quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, sogenannte "Esterquats", wie beispielsweise die unter den Warenzeichen Dehyquart^{®} und Stepantex^{®} vertriebenen Methyl-hydroxyalkyldialkoyloxyalkyl-ammoniummethosulfate, eingesetzt werden.

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Weiterhin kann es bevorzugt sein, die einzelnen Phasen mit Farbstoffen anzufärben, um ein besonders gutes optisches Erscheinungsbild des Mittels zu erzielen. Diese Farbstoffe sind bevorzugt nur in der wäßrigen oder nur in mindestens einer nichtwäßrigen Phase in einer Menge löslich, die eine entsprechende Einfärbung für den Betrachter sichtbar erscheinen läßt. Es ist auch möglich, sowohl die nichtwäßrige als auch die wäßrige Phase mit verschiedenen Farbstoffen, bevorzugt in verschiedenen Farben, einzufärben. Das alleinige Anfärben einer nichtwäßrigen Phase ist jedoch bevorzugt.

Weitere übliche Bestandteile für die erfindungsgemäßen Mittel sind:
- anionische Tenside wie beispielsweise Seifen, Alkylsulfate und Alkylpolyglykolethersulfate, Salze von Ethercarbonsäuren der Formel R-O-(CH₂-CH₂O)_{X}-CH₂-COOH, in der R eine lineare Alkylgruppe mit 10 bis 22 C-Atomen und x = 0 oder 1 bis 16 ist, Acylsarcoside, Acyltauride, Acylisethionate, Sulfobernsteinsäuremono- und dialkylester, lineare Alkansulfonate, lineare Alpha-Olefinsulfonate, alpha-Sulfofettsäuremethylester und Ester der Weinsäure und Zitronensäure Alkylglykosiden oder Alkoholen, welche Anlagerungsprodukte von etwa 2-15 Molekülen Ethylenoxid und/oder Propylenoxid an Fettalkohole mit 8 bis 22 C-Atomen darstellen.
- zwitterionische Tenside wie beispielsweise Betaine und 2-Alkyl-3-carboxymethyl-3-hydroxyethyl-imidazoline.
- ampholytische Tenside, wie beispielsweise N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren.
- nichtionische Tenside wie beispielsweise Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe, C₁₂-C₂₂-Fettsäuremono- und -diester von Anlagerungsprodukten von 1 bis 30 Mol Ethylenoxid an Glycerin, C₈-C₂₂-Alkylmono-und -oligoglycoside und deren ethoxylierte Analoga sowie Anlagerungsprodukte von 5 bis 60 Mol Ethylenoxid an Rizinusöl und gehärtetes Rizinusöl.
- nichtionische Polymere wie beispielsweise VinylpyrrolidonNinylacrylat-Copolymere, Polyvinylpyrrolidon und Vinylpyrrolidon/Vinylacetat-Copolymere,
- anionische Polymere, wie Polyacryl- und Polymethacrylsäuren, deren Salze, deren Copolymere mit Acrylsäure- und Methacrylsäureestern und -amiden und deren Derivate, die durch Kreuzvernetzung mit polyfunktionellen Agentien erhalten werden, Polyoxycarbonsäuren, wie Polyketo- und Polyaldehydocarbonsäuren und deren Salze,
   sowie Polymere und Copolymere der Crotonsäure mit Estern und Amiden der Acryl-und der Methacrylsäure, wie Vinylacetat-Crotonsäure- und Vinylacetat-Vinylpropionat-Crotonsäure-Copolymere,
- organische Verdickungsmittel wie Agar-Agar, Guar-Gum, Alginate, Celluloseether, wie Methyl- und Methylhydroxypropylcellulose, Gelatine, Pektine und/oder Xanthan-Gum. Ethoxilierte Fettalkohole, insbesondere solche mit eingeschränkter Homologenverteilung, wie sie beispielsweise als Handelsprodukt unter der Bezeichnung Arlypon^{®}F (Henkel) auf dem Markt sind, alkoxylierte Methylglucosidester, wie das Handelsprodukt Glucamate^{®} DOE 120 (Amerchol), und ethoxylierte Propylenglykolester, wie das Handelsprodukt Antil^{®} 141 (Goldschmidt), können bevorzugte organische Verdickungsmittel sein.
- Strukturanten wie Glucose und Maleinsäure,
- haarkonditionierende Verbindungen wie Phospholipide, beispielsweise Sojalecithin, Ei-Lecithin und Kephaline,
- Parfümöle,
- Lösungsvermittler, wie Ethanol, Isopropanol, Ethylenglykol, Propylenglykol, Glycerin, Diethylenglykol und ethoxylierte Triglyceride sowie Fettalkoholethoxylate und deren Derivate,
- Antischuppenwirkstoffe wie Climbazol, Piroctone Olamine und Zink Omadine,
- Wirkstoffe wie Bisabolol, Allantoin, Panthenol, Niacinmid, Tocopherol und Pflanzenextrakte,
- Lichtschutzmittel,
- Konsistenzgeber wie Zuckerester, Polyolester oder Polyolalkylether,
- Fette und Wachse, wie Walrat, Bienenwachs, Montanwachs, Paraffine, Ester, Glyceride und Fettalkohole,
- Fettsäurealkanolamide,
- Komplexbildner wie EDTA, NTA, β-Alanindiessigsäure und Phosphonsäuren,
- Quell- und Penetrationsstoffe wie PCA, Glycerin, Propylenglykolmonoethylether, Carbonate, Hydrogencarbonate, Guanidine, Harnstoffe sowie primäre, sekundäre und tertiäre Phosphate,
- Trübungsmittel wie Latex oder Styrol/Acrylamid-Copolymere,
- Perlglanzmittel wie Ethylenglykolmono- und -distearat oder PEG-3-distearat,
- direktziehende Farbstoffe sowie
- Treibmittel wie Propan-Butan-Gemische, N₂O, Dimethylether, CO₂ und Luft.

Bezüglich der weiteren Bestandteile der erfindungsgemäßen Mittel und deren übliche Einsatzmengen wird ausdrücklich auf die bekannten Monographien, z. B. Umbach, Kosmetik, 2. Auflage, Georg Thieme Verlag, Stuttgart New York, 1995, und Kh. Schrader, Grundlagen und Rezepturen der Kosmetika, 2. Auflage, Hüthig Buch Verlag, Heidelberg, 1989, verwiesen.

Die folgenden Beispiele sollen die Erfindung näher erläutern.

### Beispiele

Soweit nicht anders vermerkt, sind alle Angaben Gewichtsteile

**Wellmittel (2 Phasen)**

| | | | |
|---|---|---|---|
| | 1 (erfindungsgemäß) | 2 | 3 |
| wässrige Phase 1 | | | |
| Ammoniumthioglykolat (71%ig in Wasser) | 16,0 | 16,0 | 16,0 |
| Ammoniumhydrogencarbonat | 5,0 | 5,0 | 5,0 |
| Harnstoff | 2,0 | 2,0 | 2,0 |
| Plantapon^{®}ACG 35 ¹ | 0,5 | - | - |
| Protelan^{®} VE/K ² | - | 0,6 | - |
| Merquat^{®}100 ³ | 0,2 | 0,2 | 0,2 |
| Turpinal^{®} SL ⁴ | 0,3 | 0,3 | 0,3 |
| 2-Ethylhexandiol-1,3 | 0,5 | 0,5 | 0,5 |
| Ammoniak (25 %ig in Wasser) | 3,0 | 3,0 | 3,0 |
| Wasser | 62,5 | 62,4 | 63,0 |
| Ölphase 2 | | | |
| Parfümöl | 1,0 | 1,0 | 1,0 |
| Wacker Belsil^{®} ADM 652 ⁵ | 1,0 | 1,0 | 1,0 |
| Dow Corning^{®} 345 Fluid ⁶ | 8,0 | 8,0 | 8,0 |

| | | | |
|---|---|---|---|
| ¹ Dinatrium N-Kokoyl-glutamat (31 % Aktivsubstanz; INCI-Bezeichnung: Sodium Cocoyl Glutamate) (Cognis Deutschland) ² Weizenproteinhydrolysat-Kokosfettsäure-Kondensat, Natriumsalz (26 % Aktivsubstanz in Wasser; INCI-Bezeichnung: Sodium Cocoyl Hydrolyzed Wheat Protein) (Zschimmer & Schwarz) ³ Poly(dimethyldiallylammoniumchlorid) (40 % Aktivsubstanz; INCI-Bezeichnung: Polyquaternium-6) (Ondeo Nalco) ⁴ 1-Hydroxyethan-1,1-diphosphonsäure (30% Aktivsubstanz in Wasser; INCI-Bezeichnung: Etidronic Acid) (Henkel) ⁵ Polydimethylsiloxan mit Aminoalkylgruppen (INCI-Bezeichnung: Amodimethicone) (Wacker) ⁶ Decamethylcyclopentasiloxan (INCI-Bezeichnung: Cyclomethicone) (Dow-Corning) | | | |

Die Wellmittel 1 bis 3 wurden in je einen Zylinder aus Glas gefüllt. Dann wurde jeder Zylinder 5 Misch-Entmisch-Zyklen unterworfen. Pro Zyklus wurde jedes Wellmittel in dem Zylinder 25 mal geschüttelt und anschließend zur Entmischung 5 Minuten ruhen gelassen. Nach dem fünften und letzten Mischvorgang wurde zur Entmischung 30 Minuten ruhen gelassen. Das Misch- und Entmischungsverhalten der Wellmittel wurde begutachtet.

Das erfindungsgemäße 2-phasige Wellmittel 1 ließ sich durch 5 Misch-Entmisch-Zyklen stets in eine homogene Mischung umwandeln und besitzt daher ein hervorragendes Entmischungsverhalten unter Rückbildung des Ausgangszustandes.

Das nicht erfindungsgemäße 2-phasige Wellmittel 2 ließ sich zwar in den 5 Misch-Entmisch-Zyklen in eine homogene Mischung umwandeln, es fand jedoch eine unvollständige Phasentrennung statt. Der Ausgangszustand des mehrphasigen Wellmittels 2 wurde somit nicht wieder hergestellt.

Das nicht erfindungsgemäße Wellmittel 3 ließ sich nicht in eine homogene Mischung überführen.

## Patentansprüche

1. Mittel zur Durchführung der reduzierenden Stufe eines Verfahrens zur dauerhaften Verformung von Keratinfasern enthaltend eine keratinreduzierende Substanz sowie übliche Bestandteile, **dadurch gekennzeichnet, daß** es in Form eines Zwei- oder Mehrphasensystems, enthaltend mindestens zwei separate, kontinuierliche Phasen, vorliegt, das mindestens eine Ölkomponente und als Emulgator mindestens eine N-Acyl-glutaminsäure oder deren Salze enthält und das durch mechanische Bewegung reversibel in ein homogenes System überführt werden kann.

2. Mittel zum Spülen nach Durchführung der reduzierenden Stufe eines Verfahrens zur dauerhaften Verformung von Keratinfasern enthaltend übliche Bestandteile, **dadurch gekennzeichnet, daß** es in Form eines Zwei- oder Mehrphasensystems, enthaltend mindestens zwei separate, kontinuierliche Phasen, vorliegt, das mindestens eine Ölkomponente und als Emulgator mindestens eine N-Acyl-glutaminsäure oder deren Salze enthält und das durch mechanische Bewegung reversibel in ein homogenes System überführt werden kann.

3. Mittel zur Durchführung der oxidierenden Stufe eines Verfahrens zur dauerhaften Verformung von Keratinfasern enthaltend ein Oxidationsmittel sowie übliche Bestandteile, **dadurch gekennzeichnet, daß** es in Form eines Zwei- oder Mehrphasensystems,enthaltend mindestens zwei separate, Kontinuiertiche Phasen, vorliegt, das mindestens eine Ölkomponente und als Emulgator mindestens eine N-Acyl-glutaminsäure oder deren Salze enthält und das durch mechanische Bewegung reversibel in ein homogenes System überführt werden kann.

4. Mittel nach einem der Ansprüche 1 bis 3, **dadurch gekennzeichnet, daß** die N-Acyl-glutaminsäure ausgewählt ist aus einer N-(C₈ bis C₃₀)-Acyl-glutaminsäure oder deren Salzen.

5. Mittel nach einem der Ansprüche 1 bis 4, **dadurch gekennzeichnet, daß** die Ölkomponente ausgewählt ist aus pflanzlichen Ölen, Paraffinölen und Silikonen.

6. Mittel nach einem der Ansprüche 1 bis 5, **dadurch gekennzeichnet, daß** zusätzlich ein mit Wasser begrenzt mischbarer Alkohol enthalten ist, der in Wasser bei 20°C zu nicht mehr als 10 Gew.-% bezogen auf die Wassermasse löslich ist.

7. Mittel nach Anspruch 6, **dadurch gekennzeichnet, daß** der mit Wasser begrenzt mischbare Alkohol 4 bis 20 Kohlenstoffatome aufweist.

8. Mittel nach einem der Ansprüche 1 bis 7, **dadurch gekennzeichnet, daß** ein konditionierender Wirkstoff enthalten ist.

9. Verfahren zur dauerhaften Verformung von Keratinfasern, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült und gegebenenfalls nachbehandelt, **dadurch gekennzeichnet, daß** mindestens eine der beiden wäßrigen Zubereitungen oder die erste Spülung in Form eines Zwei- oder Mehrphasensystems,enthaltend mindestens zwei separate, Kontinuierliche Phasen, vorliegt, das mindestens eine Ölkomponente und mindestens eine N-Acyl-glutaminsäure oder deren Salze enthält und das zum Aufbringen auf die Faser durch mechanische Bewegung reversibel in ein homogenes System überführt wird.

10. Verfahren nach Anspruch 9, **dadurch gekennzeichnet, daß** die Ölkomponente ausgewählt ist aus pflanzlichen Ölen, Paraffinölen und Silikonen.

11. Verfahren nach einem der Ansprüche 9 oder 10, **dadurch gekennzeichnet, daß** die N-Acyl-glutaminsäure ausgewählt ist aus einer N-(C₈ bis C₃₀)-Acyl-glutaminsäure oder deren Salzen.

12. Verfahren nach einem der Ansprüche 9 bis 11, **dadurch gekennzeichnet, daß** zusätzlich ein mit Wasser begrenzt mischbarer Alkohol enthalten ist.

13. Verfahren nach Anspruch 12, **dadurch gekennzeichnet, daß** der mit Wasser begrenzt mischbare Alkohol 4 bis 20 Kohlenstoffatome aufweist.

## Claims

1. An agent for performing the reducing stage of a method for permanently shaping keratin fibres, containing a keratin-reducing substance as well as conventional constituents, **characterised in that** said agent assumes the form of a two- or multi-phase system, containing at least two separate, continuous phases, which system contains at least one oil component and, as emulsifier, at least one N-acyl glutamic acid or the salts thereof and which may be converted reversibly by mechanical movement into a homogeneous system.

2. An agent for rinsing after performing the reducing stage of a method for permanently shaping keratin fibres, containing conventional constituents, **characterised in that** said agent assumes the form of a two- or multi-phase system, containing at least two separate, continuous phases, which system contains at least one oil component and, as emulsifier, at least one N-acyl glutamic acid or the salts thereof and which may be converted reversibly by mechanical movement into a homogeneous system.

3. An agent for performing the oxidising stage of a method for permanently shaping keratin fibres, containing an oxidising agent as well as conventional constituents, **characterised in that** said agent assumes the form of a two- or multi-phase system, containing at least two separate, continuous phases, which system contains at least one oil component and, as emulsifier, at least one N-acyl glutamic acid or the salts thereof and which may be converted reversibly by mechanical movement into a homogeneous system.

4. An agent according to any one of claims 1 to 3, **characterised in that** the N-acyl glutamic acid is selected from an N-(C₈ to C₃₀)-acyl glutamic acid or the salts thereof.

5. An agent according to any one of claims 1 to 4, **characterised in that** the oil component is selected from vegetable oils, paraffin oils and silicones.

6. An agent according to any one of claims 1 to 5, **characterised in that** an alcohol miscible to a limited extent with water is additionally contained therein, said alcohol being no more than 10 wt.% soluble in water at 20°C relative to the mass of water.

7. An agent according to claim 6, **characterised in that** the alcohol miscible to a limited extent with water comprises 4 to 20 carbon atoms.

8. An agent according to any one of claims 1 to 7, **characterised in that** a conditioning active ingredient is contained therein.

9. A method for permanently shaping keratin fibres, in which the fibres are treated before and/or after mechanical shaping with an aqueous preparation of a keratin-reducing substance, rinsed after a period of exposure with a first rinse, then fixed with an aqueous preparation of an oxidising agent and rinsed again after a period of exposure and optionally post-treated, **characterised in that** at least one of the two aqueous preparations or the first rinse assumes the form of a two- or multi-phase system, containing at least two separate, continuous phases, which system contains at least one oil component and at least one N-acyl glutamic acid or the salts thereof and which may be converted reversibly by mechanical movement into a homogeneous system for application onto the fibres.

10. A method according to claim 9, **characterised in that** the oil component is selected from vegetable oils, paraffin oils and silicones.

11. A method according to either one of claims 9 or 10, **characterised in that** the N-acyl glutamic acid is selected from an N-(C₈ to C₃₀)-acyl glutamic acid or the salts thereof.

12. A method according to any one of claims 9 to 11, **characterised in that** an alcohol miscible to a limited extent with water is additionally contained therein.

13. A method according to claim 12, **characterised in that** the alcohol miscible to a limited extent with water comprises 4 to 20 carbon atoms.

## Revendications

1. Agent pour la mise en oeuvre de l'étape de réduction d'un procédé pour le permanentage de fibres kératiniques contenant une substance de réduction de la kératine ainsi que des constituants habituels, **caractérisé en ce qu'**il est présent sous la forme d'un système biphasique ou polyphasique contenant au moins deux phases continues séparées, qui contient au moins un composant huileux et à titre d'émulsifiant au moins un acide N-acyl-glutamique ou ses sels, et qui peut être transformé de manière réversible, via un mouvement mécanique, en un système homogène.

2. Agent de rinçage, après le franchissement de l'étape de réduction d'un procédé pour le permanentage de fibres kératiniques contenant des constituants habituels, **caractérisé en ce qu'**il est présent sous la forme d'un système biphasique ou polyphasique contenant au moins deux phases continues séparées, qui contient au moins un composant huileux et à titre d'émulsifiant au moins un acide N-acyl-glutamique ou ses sels, et qui peut être transformé de manière réversible, via un mouvement mécanique, en un système homogène.

3. Agent pour la mise en oeuvre de l'étape d'oxydation d'un procédé pour le permanentage de fibres kératiniques, contenant un agent d'oxydation ainsi que des constituants habituels, **caractérisé en ce qu'**il est présent sous la forme d'un système biphasique ou polyphasique contenant au moins deux phases continues séparées, qui contient au moins un composant huileux et à titre d'émulsifiant au moins un acide N-acyl-glutamique ou ses sels, et qui peut être transformé de manière réversible, via un mouvement mécanique, en un système homogène.

4. Agent selon l'une quelconque des revendications 1 à 3, **caractérisé en ce que** l'acide N-acyl-glutamique est choisi parmi un acide N-acyl(en C₈-C₃₀)-glutamique ou ses sels.

5. Agent selon l'une quelconque des revendications 1 à 4, **caractérisé en ce que** le composant huileux est choisi parmi des huiles végétales, des huiles paraffiniques et des silicones.

6. Agent selon l'une quelconque des revendications 1 à 5, **caractérisé en ce qu'**il contient en outre un alcool manifestant une miscibilité limitée avec l'eau, qui est soluble dans l'eau à 20 °C à concurrence d'une valeur qui n'est pas supérieure à 10 % en poids, rapportée à la masse d'eau.

7. Agent selon la revendication 6, **caractérisé en ce que** l'alcool manifestant une miscibilité limitée avec l'eau présente de 4 à 20 atomes de carbone.

8. Agent selon l'une quelconque des revendications 1 à 7, **caractérisé en ce qu'**il contient une substance active de revitalisation.

9. Procédé pour le permanentage de fibres kératiniques, dans lequel on traite les fibres avant et/ou après une déformation mécanique avec une préparation aqueuse d'une substance de réduction de la kératine; après avoir laissé agir pendant un certain temps, on procède à un premier rinçage; on fixe ensuite avec une préparation aqueuse d'un agent d'oxydation et, également après avoir laissé agir pendant un certain temps, on rince et on procède le cas échéant à un traitement ultérieur, **caractérisé en ce qu'**au moins une des deux préparations aqueuses ou le premier rinçage est présent sous la forme d'un système biphasique ou polyphasique contenant au moins deux phases continues séparées, qui contient au moins un composant huileux et à titre d'émulsifiant au moins un acide N-acyl-glutamique ou ses sels, et qui est transformé, à des fins d'application sur les fibres, de manière réversible, via un mouvement mécanique, en un système homogène.

10. Procédé selon la revendication 9, **caractérisé en ce que** le composant huileux est choisi parmi des huiles végétales, des huiles paraffiniques et des silicones.

11. Procédé selon l'une quelconque des revendications 9 ou 10, **caractérisé en ce que** l'acide N-acyl-glutamique est choisi parmi un acide N-acyl(en C₈-C₃₀)-glutamique ou ses sels.

12. Procédé selon l'une quelconque des revendications 9 à 11, **caractérisé en ce que** la préparation contient en outre un alcool manifestant une miscibilité limitée avec l'eau.

13. Procédé selon la revendication 12, **caractérisé en ce que** l'alcool manifestant une miscibilité limitée avec l'eau présente de 4 à 20 atomes de carbone.
